# EUROPEAN PATENT SPECIFICATION

(11) **EP 3 599 897 B1**
(45) Date of publication and mention of the grant of the patent: **11.05.2022**
(21) Application number: 18715980.1
(22) Date of filing: 28.03.2018
(51) Int. Cl.: A23L 33/105, A61K 36/185, A23L 33/185

(54) **METHOD FOR REALIZATION OF DRIED EXTRACTS DERIVED OF LEAVES AND PLANT SEEDS OF MORINGA OLEIFERA.**
VERFAHREN ZUR HERSTELLUNG VON GETROCKNETEN EXTRAKTEN AUS BLÄTTERN UND PFLANZENSAMEN VON MORINGA OLEIFERA
PROCÉDÉ D'OBTENTION D'EXTRAITS SECS DÉRIVÉS DE FEUILLES ET DE GOUSSES DE BEN OLÉIFÈRE

(30) Priority: 30.03.2017 EP 17163801
(43) Date of publication of application: 05.02.2020
(73) Proprietor: Specchiasol S.r.l., 37012 Bussolengo (VR) (IT)
(72) Inventor: SCIALPI, Antonio, 37012 Bussolengo (VR) (IT)
(74) Representative: Petraz, Gilberto Luigi
(86) International application number: PCT/IB2018/052112
(87) International publication number: WO 2018/178886

(56) References cited:
- CN-A- 102 920 754
- CN-A- 104 997 817
- CELIA RODRÍGUEZ-PÉREZ ET AL: "Optimization of microwave-assisted extraction and pressurized liquid extraction of phenolic compounds from Moringa oleifera leaves by multiresponse surface methodology", ELECTROPHORESIS, vol. 37, no. 13, 1 July 2016 (2016-07-01), pages 1938-1946, XP055473228, ISSN: 0173-0835, DOI: 10.1002/elps.201600071
- PREMI MONICA ET AL: "Effect of extraction conditions on the bioactive compounds from Moringa oleifera(PKM 1) seeds and their identification using LC-MS", JOURNAL OF FOOD MEASUREMENT AND CHARACTERIZATION, SPRINGER US, BOSTON, vol. 11, no. 1, 10 August 2016 (2016-08-10), pages 213-225, XP036160817, ISSN: 2193-4126, DOI: 10.1007/S11694-016-9388-Y [retrieved on 2016-08-10]
- SIDDHURAJU P ET AL: "Antioxidant properties of various solvent extracts of total phenolic constituents from three different agroclimatic origins of drumstick tree (Moringa oleifera Lam.) leaves", JOURNAL OF AGRICULTURAL AND FOOD CHEMISTRY, AMERICAN CHEMICAL SOCIETY, BOOKS AND JOURNALS DIVISION, US, vol. 51, no. 8, 9 April 2003 (2003-04-09), pages 2144-2155, XP002718067, ISSN: 0021-8561, DOI: 10.1021/JF020444+ [retrieved on 2003-03-07]
- FÖRSTER NADJA ET AL: "Development of a reliable extraction and quantification method for glucosinolates inMoringa oleifera", FOOD CHEMISTRY, ELSEVIER LTD, NL, vol. 166, 14 June 2014 (2014-06-14), pages 456-464, XP029039726, ISSN: 0308-8146, DOI: 10.1016/J.FOODCHEM.2014.06.043

## Description

The present invention relates to a method for realization dried extracts, hydro-alcoholic extracts and protein powders derived of leaves and plant seeds of Moringa oleifera.

In particular, the invention in object covers the realization of dry extracts, hydroalcoholic extracts and powders derived from the plant Moringa oleifera with known content of glucosinolates, polyphenols and proteins.

CELIA RODRíGUEZ-PÉREZ ET AL,ELECTROPHORESIS, vol. 37, no. 13, 1 July 2016, pages 1938-1946, relates to optimization of microwave-assisted extraction and pressurized liquid extraction of phenolic compounds from Moringa oleifera leaves by multiresponse surface methodology.

### Scope of the Invention.

The invention involves the realization of extracts of *Moringa oleifera* (Moringa) standardized in bioactive components such as Glucosinolates (GLNs) or Polyphenols (PPs) and to be used for the production of nutraceuticals, especially having good potential as nutritional supplements or like.

The present invention is defined in the appended claims.

### Description.

1. Moringa oleifera Lam. (Moringa) belongs to the family Moringaceae in phylogeny tree. It is widely distributed and known native in India, Afghanistan, Bangladesh, and Pakistan (Amaglo et al., 2010).

Moringa is also called with various names such as horseradish tree, drumstick tree and locally named 'murungai' or 'kelor'.

The plant is known growing in arid environment such West, East, South Africa, Tropical Asia and Latin America.

The plant is edible and consumed by most of the people in Asia including Thailand, Malaysia and India (Ghosh, 2013).

Moringa is a perennial plant, has spirally arranged leaves, whitish flower and low quality of timber and with the following characteristics:
Order: Brassicales
Family: Moringaceae
Genus: Moringa
Species: Moringa oleifera
Binomial name: Moringa oleifera Lam.

Seeds, leaves, oil, sap, bark, roots, and flowers are widely used in traditional medicine. Moringa leaves have been characterized to contain nutritional factors such as vitamins, minerals, amino acids and fatty acids (Teixeira et al., 2014). Additionally, the leaves contain various types of antioxidant compounds such as ascorbic acid, flavonoids, phenolics and carotenoids (Vongsak et al., 2014), while seed contain mainly glucosinolates.

According to several authors (Razis et al., 2014), various preparations of *Moringa oleifera* are used for their anti-inflammatory, antihypertensive, diuretic, antimicrobial, antioxidant, antidiabetic, antihyperlipidemic, antineoplastic, antipyretic, antiulcer, cardio- and hepato-protectant activities. The therapeutic potential of M. *oleifera* leaves in treating hyperglycemia and dyslipidemia was reviewed by Mbikay (2012). Razis et al. (2014) summarized potential health benefits of Moringa oleifera, focusing on their nutritional content as well as antioxidant and antimicrobial characteristics.

The main flavonoids found in leaves are glycosylated derivatives of quercetin and kaempferol (see Figure 1 below), whereas the seed has higher amount of glucosinolates.

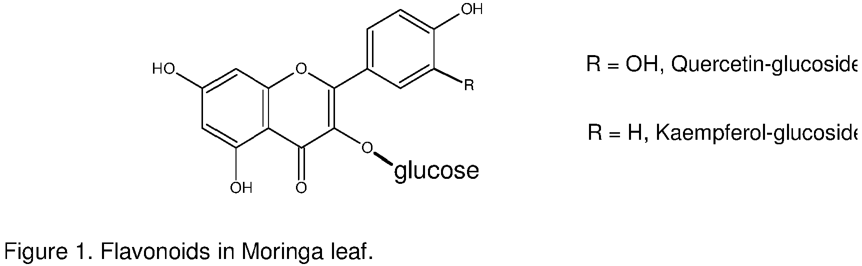

Glucosinolates are the secondary metabolites typical of Brassicales plant such as M. *oleifera* (Maldini et al., 2014). Moringa contains high amount of aromatic glucosinolates (see Figure 2 below) such as p-hydroxybenzyl-glucosinolate (sinalbin), 2-phenylethyl-glucosinolat (gluconasturtiin) and benzyl glucosinolate (glucotropaeolin) (Forster et al., 2015).

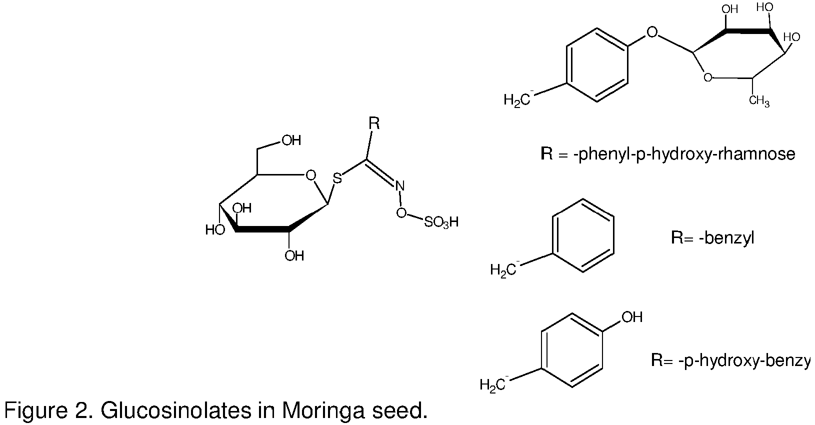

The amount of glucosinolates found in the seeds was approximately 8%, which was the highest value compared to other parts of the plant. Glucosinolates were reported to induce apoptosis in cancer cell and the major studies had been done on leaf extracts (Forster et al., 2015). Studies on the anti-cancer of the seed extract are very scarce, thus it is important to carry out anti-cancer studies of the seed due to the abundance of glucosinolates presented in the seeds.

Table 1 below shows some reports regarding *in vitro* studies of M. *oleifera* for potential chemoprevention agent.

**Table 1. Reports on cancer chemoprevention of Moringa oleifera.**

| Study | Remarks | Reference |
|---|---|---|
| Anti-proliferation and induction of apoptosis by Monuga oleifera leaf extract on human cancer cells | Extracts induced apoptosis in human tumor cell line | Sreelatha et al., (2011) |
| Ethanolic extract of Monuga oleifera increased cytotoxic effect of doxorubicin on HeLa cancer cells | Combination of extract and doxombicin increased doxombicin effect throngh apoptotic induction | Hermawan et al., (2012) |
| Antioxidant and anticancer oleifera leaves | Dichloromethane extract showed high antioxidant and potent anti -cancer proliferation | Ctharoensin (2014) |
| Moringa species (Moringaceae): phytochemistry, cancer chemoprevention potentials with advanced traditional medicinal practice | Skin tumor prevention and exhibit good hepatoprotective | Dibyajyoti (2013) |
| Anticancer effect of Moringa oleifin leaf extract on human breast cancer cell | Anti-proliferative effect on 2 breast cancer cell lines, MDA MB 231 and MCF 7, showed a dose and dependent effect | Ghosh (2013) |

| | | |
|---|---|---|
| From Karim et al., 2016. | | |

The uses of *Moringa oleifera* standardized extract as commercial ingredient has not been developed yet.

However, it showed positive synergistic effect in increasing cytotoxicity of doxorubicin against cervical cancer cell line (Adam et al., 2012).

The combination of *Moringa oleifera* and doxorubicin were resulted to enhance apoptosis induction. Interestingly, the author also stated that the mechanisms involved by the combination of *Moringa oleifera* and doxorubicin were still unclear.

Both chemical compounds may have ability to induce apoptosis, so when both were combined, they exhibited stronger effect.

The findings of the study suggested that by combining *Moringa oleifera* extract to cancer therapy drug available, it may decrease toxic side effect of the drug as well as enhancing chemopreventive effect of the drug.

*Moringa oleifera* extracts had long been used in traditional medicine.

This proved that the plant is safe to consume and taken as edible vegetable.

### - Moringa oleifera - Safety

No adverse effects were reported in any of the human studies that have been conducted to date. Furthermore, various preparations have been and continued to be used around the world as foods and as medicinals without the report of ill effects.

Several animal studies have assessed the potential toxicity of various preparations on *Moringa oleifera.* For example, the safety of an aqueous leaf extract given orally to rats at doses in the range 0.4-2 g/kg body weight was examined (Adedapo et al., 2009).

The treatment was either an acute single dose or given daily for 21 days except the highest dose. Various parameters were assessed including blood cell counts and serum enzyme levels.

The authors concluded that consumption of Moringa leaves at doses of up to 2 mg/kg were safe. Bakre et al. (2013) determined that the LD50 an orally administered ethanol extract of M. oleifera leaves in mice was greater than 6.4 g/kg.

In addition, other studies reported the safety of the Moringa are reported in literature and recently reviewed by Stohs and Hartman (2015).

### 2. Experimental part

### 2.1 Materials

The compounds glucosinalbin and quercetin-3-glucoside were provided by Extrasynthese (Genay,F). Methanol, acetonitrile and formic acid were purchased from Sigma-Aldrich (St. Louis, MO, USA).

### 2.2 Sample preparation for glucosinolate identification and quantification

*Moringa oleiferaseeds* and leaves were ground to a fine powder and extracted with aqueous solution containing ethanol in the range 0-100% (sample to solvent ratio 1:40 w/v). The mixtures were kept at T.A. for 20 min under vortex mixing. The samples were successively centrifuged at 1000 × g for 10 minand the supernatants were collected and stored at 4°C.

### 2.3 Glucosinolate identification and quantification

The qualitative analysis was carried out on an Acquity UHPLC separation module (Waters, Milford, MA, USA) coupled with amod. E-lambda diode-array detector (Waters) and a model Exactive Orbitrap MS equipped with an HESI-II probe for electrospray ionization (Thermo Scientific, San Jose, CA, USA) set in negative ion mode.The ion source and interface conditions were: spray voltage -3.0 kV, capillary -72 V, tube lens -170 V, skimmer -23 V, sheath gas flow-rate 45, auxiliary gas flow-rate 10, heater and capillary temperature 280 and 150 °C, respectively. A 1.8 µm HSS T3 column (150x2.1 mm, Waters) was used for separation at a flow-rate of 0.45 ml/min. The eluents were 0.05% HCOOH in MilliQ-treated water (solvent A) and CH₃CN (solvent B). Five µl of the sample were separated by the UHPLC using the following elution gradient: 0% B for 4 min, 0-20% B in 20 min, 20-80 % B in 5 min and then return to initial conditions in 1 min. The column and samples were kept at 50 and 20 °C, respectively. The UHPLC eluate was analyzed in full scan MS in the range *(m*/*z)⁻* 50-1000 u. The resolution, AGC target, maximum ion injection time and mass tolerance were 50 K, 1 E6, 100 ms and 2 ppm, respectively. The HCD was opened and the applied voltages were 15 and 30eV. The MS data were processed using Xcalibur software (Thermo Scientific).

The quantitative analysis was carried out on an Alliance mod. 2695 (Waters, Milford, MA) equipped with a mod. 2996 (Waters) photodiode array detector. A 5µm C₁₈ Atlantis T3 column (250x4.6 mm, Waters) was used for the separation, which was performed by means of a linear gradient elution (eluent A, 0.1% formic acid; eluent B, acetonitrile) at a flow rate of 1.5mL/min. The gradient was as follows: 0% B for 2 min, 0 to 10% B for 10 min, 10 to 90% B in 5 min and then 90% B for 2 min. The column and sample was maintained at 30 and 20°C, respectively. Twenty µL was injected in the chromatographic system. Data were acquired in the 195-350 nm range and integrated at 205 nm. Calibration curve was obtained from glucosinalbin stock solutions prepared by dissolving 20 mg of standard powder in 20 mL of methanol. The working solutions in water were in the range of 10-500 µg/mL.

### 2.4 Flavonoid qualitative and quantitative determination

The qualitative analysis was carried out on an Acquity UHPLC separation module (Waters, Milford, MA, USA) coupled with amod. E-lambda diode-array detector (Waters) and a model Exactive Orbitrap MS equipped with an HESI-II probe for electrospray ionization (Thermo Scientific, San Jose, CA, USA) set in negative ion mode.The ion source and interface conditions were: spray voltage -3.0 kV, capillary -72 V, tube lens -170 V, skimmer -23 V, sheath gas flow-rate 45, auxiliary gas flow-rate 10, heater and capillary temperature 350 and 150 °C, respectively. A 1.7 µm BEH Shied C₁₈ column (150x2.1 mm, Waters) was used for separation at a flow-rate of 0.45 ml/min. The eluents were 0.1% HCOOH in MilliQ-treated water (solvent A) and CH₃CN (solvent B). Five µl of the sample were separated by the UHPLC using the following elution gradient: 2% B for 2 min, 2-85% B in 20 min, 85 % B for 5 min and then return to initial conditions in 1 min. The column and samples were kept at 50 and 20 °C, respectively. The UHPLC eluate was analyzed in full scan MS in the range *(m*/*z)⁻* 50-1000 u. The resolution, AGC target, maximum ion injection time and mass tolerance were 50 K, 1 E6, 100 ms and 2 ppm, respectively. The HCD was opened and the applied voltages were 15 and 30eV. Spectral data were acquired in the range 200-450 nm and integrated at 354 nm. The data were processed using Xcalibur software (Thermo Scientific).

The quantitative analysis was carried out on achromatographic system model Alliance 2695 (Waters) equipped with a mod. 2996 (Waters) photodiode array detector. A 3.5µm C₁₈ Symmetry column (150x2.1 mm, Waters) was used for the separation, which was performed by means of a linear gradient elution (eluent A, 0.1% formic acid; eluent B, acetonitrile) at a flow rate of 0.35mL/min. The gradient was as follows: from 10 to 30% B for 10 min, 10 to 90% B in 5 min and then 90% B for 2 min. The column and sample was maintained at 30 and 20°C, respectively. Five µL was injected in the chromatographic system. Data were acquired in the range 200-450 nm and integrated at 354 nm. Calibration curve was obtained from quercetin-glucoside stock solutions prepared by dissolving 20 mg of standard powder in 20 mL of methanol. The working solutions in methanol were in the range of 10-100 µg/mL.

### 2.5 Protein analysis

*Moringa oleifera* leaves contain aminoacids in higher levels than those recommended by theFood and Agriculture Organization (WHO-FAO), withpatterns similar to those of soybean seeds. Moreover, analyses of the composition ofM. *oleifera* seeds and leaves have showed levels of proteins in the range 33-38% and 26-29%, respectively. Thus, the amount of protein found in seed and leaf of Moringa was higher than that present in important legumes present in the human diet.

The nitrogen content was determined by conventional acid hydrolysis and Kjeldahl digestion using a copper catalyst in 2 g of dried leaves andseeds. Moreover, protein was determined also in the dried residue of the extraction of glucosinolates or polyphenols.

The ammonia was distilled and collected in a solution of boric acid which was then titrated against standard acid. Digestion and distillation were carried out using the Kjeltec apparatus (Model 1002, Tecator, Sweden). Protein content was calculated as total N × 6.25.

### 2.6 Extraction and stability test

Moringa seeds were dissolved in water and the sample to solvent ratio wasin the range 1:5 - 1:200w/v.The mixture was sonicated for 140 min and at fixed times 50 µl was withdraw, diluted to 1 ml by water and the solutions centrifuged at 1000 × g for 90 sec. 20 µL of the clear solution was injected in the HPLC system.

### 3. Results

### 3.1 Glucosinolates in Moringa seeds.

The full negative mass spectrum recorded for seed pulp extract (see Fig. 3 below) revealed a major intense ion peak at *m*/*z* of 570.0953u and a second less intensive peak with *m*/*z*390.0537 u. Fragmentation experiments were performed with the aim to ascertain the nature of these compounds. Regarding the ion with m/zof 570.0953 u, product ions spectrum (see Fig. 4 below) evidenced one major ion peak at *m*/*z* value of 96.9604 corresponding to deprotonated ion of sulphate. Fragmentation of the ion with *m*/*z* 570.095 u (see Fig. 5 below) displayed also fragment ions at values of m/zof 259.0128 u. The ion with *m*/*z* of 570.0953 u, with formula brute, C20H2₉NOi4S2, has been identified as glucosinalbin-rhamnoside.

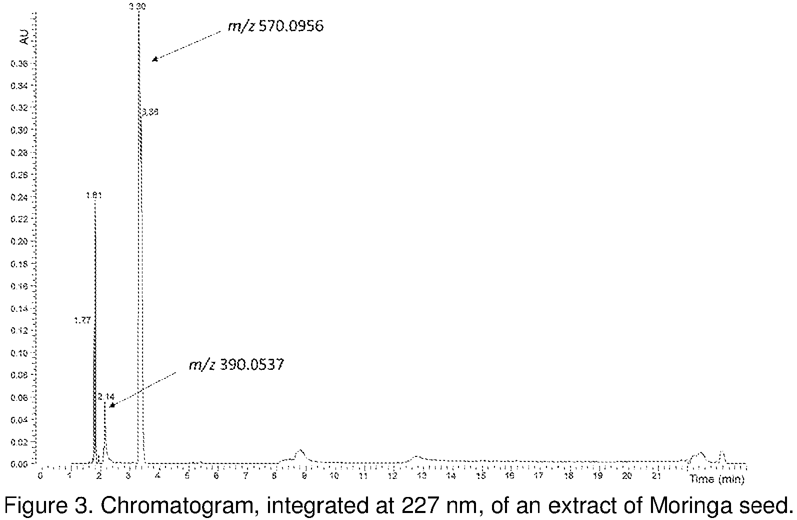

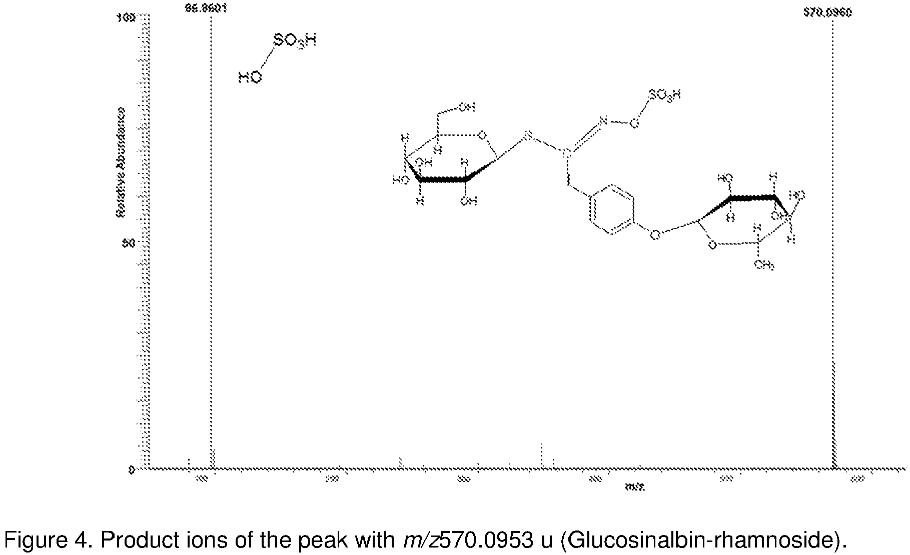

Regarding the ion with m/zof 390.0537 u, product ions spectrum (Fig. 5) evidenced one major ion peak at *m*/*z* value of 96.9604 corresponding to deprotonated ion of sulphate. Thus, the ion with *m*/*z* of 390.0537 u, with formula brute, C₁₁H21NO₁₀S₂, has been identified as Glucoconringiin.

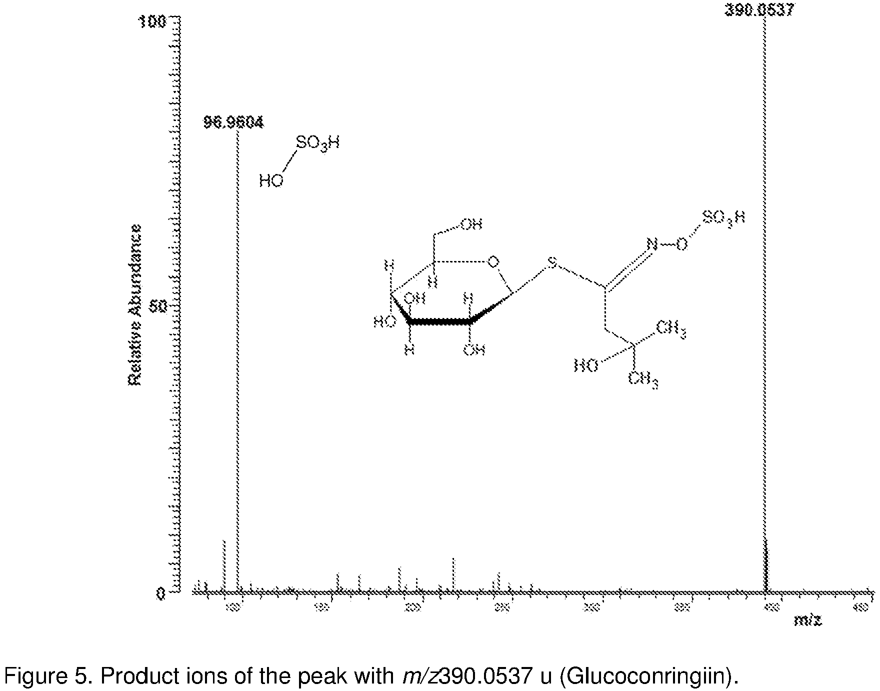

### 3.2 Quantitative determination of the glucosinolates

Sinalbin working solutions were in the range 8-400 µg/mL and the calibration curve is reported in figure 6. The rhamnosyl-sinalbin percentage in the analyzed samples is reported in table 2.

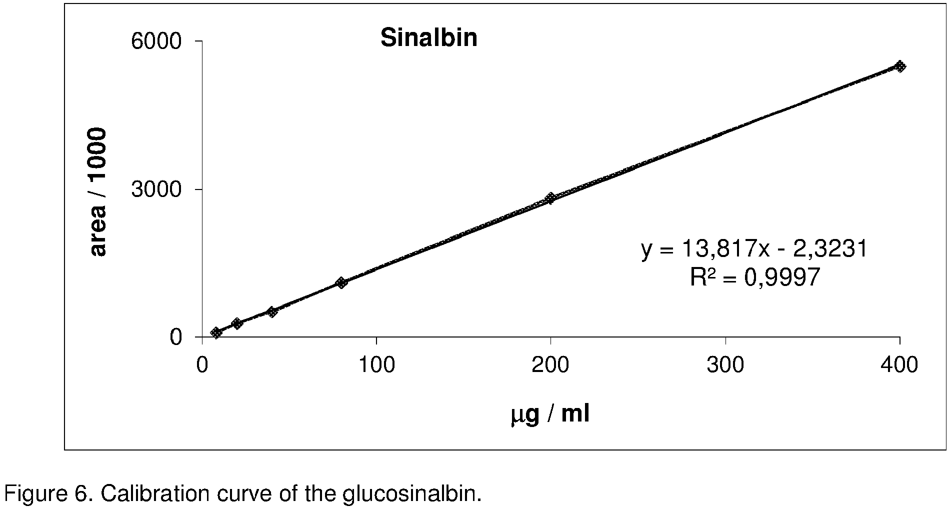

**Table 2. Glucosinolate percentage in the samples of Moringa seeds.**

| **Sample** | **Description** | **Glucosinolates (%)** |
|---|---|---|
| 1 | Seeds (Powder) | 4.5-5.0 |
| 2 | Seeds | 7.0-8.0 |
| 3 | Seeds (Powder) | 4.5-5.2 |
| 4-12 | Leaves (micronized) | n.f. |

### 3.3 Recovery of Glucosinolates in Moringa seed extracts

Moringa seeds were extracted with aqueous solutions containing 0-20-40-60-80 and 100% ethanol and results are showed in figure 7. It seems that the solutions containing water or low percentages of ethanol (e.g. 20%, v/v) were effective in extracting quantitatively glucosinolates from seeds.

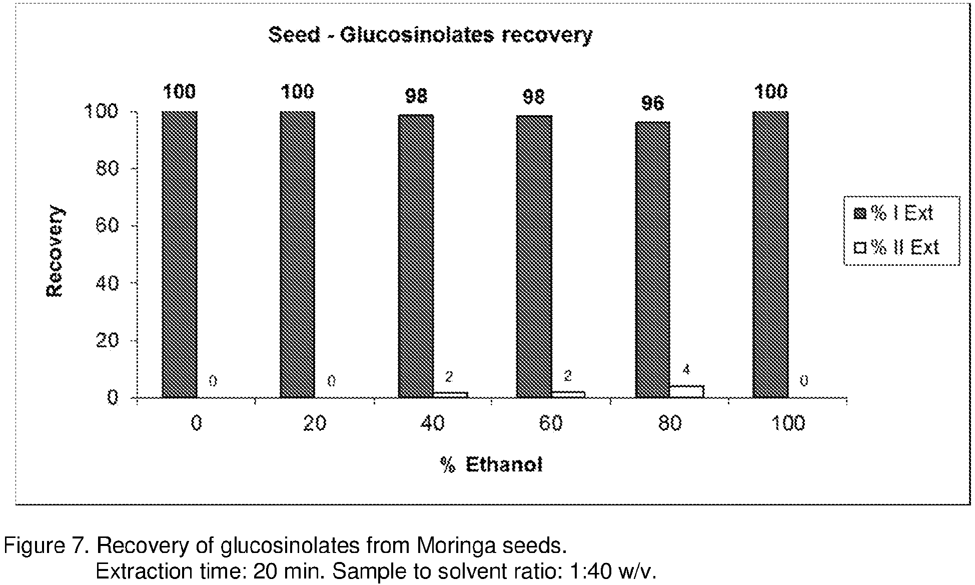

Moringa seeds were dissolved in water, sample to solvent ratio: 1:5 w/v, and the mixture was sonicated for 140 min. At fixed times aliquote of 50 µl was withdraw, diluted to 1 ml by water and the solutions centrifuged at 1000 g for 90 sec. 20 µL of the clear solution was injected in the HPLC system. Results are showed in figure 8. It seems that in the applied conditions the glucosinolate was not stable. Indeed, after 60 min about 63% of the glucosinolate disappeared.

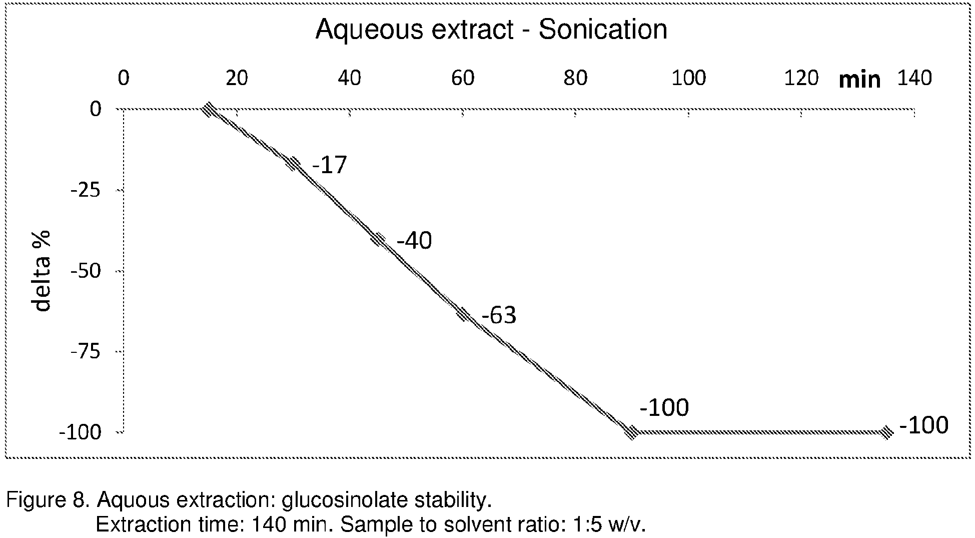

To establish the effect of the sonication, Moringa seeds dissolved in water, sample to solvent ratio: 1:5 w/v, were extracted for 65 min by a vertical vibration system. At fixed times aliquote of 50 µl was withdraw, diluted to 1 ml by water and the solutions centrifuged at 1000 g for 90 sec. 20 µL of the clear solution was injected in the HPLC system. Results are showed in figure 9. It seems that in the applied conditions the glucosinolate was more stable than extract obtained by sonication. Indeed, after 60 min about 36% of the glucosinolate disappeared.

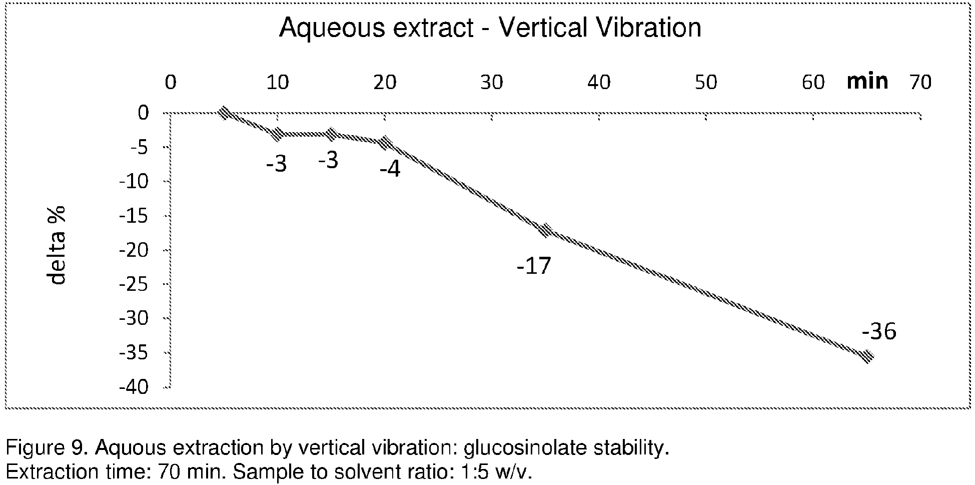

To establish the effect of the organic solvent, Moringa seeds dissolved in a aqueous solution containing 20% ethanol, sample to solvent ratio: 1:5 w/v, were extracted for 70 min by a vertical vibration system. At fixed times aliquote of 50 µl was withdraw, diluted to 1 ml by water and the solutions centrifuged at 1000 g for 90 sec. 20 µL of the clear solution was injected in the HPLC system. Results are showed in figure 10. It seems that in the applied conditions the glucosinolate was more stable than extract obtained using water as solvent. Indeed, after 60 min about 10% of the glucosinolate disappeared.

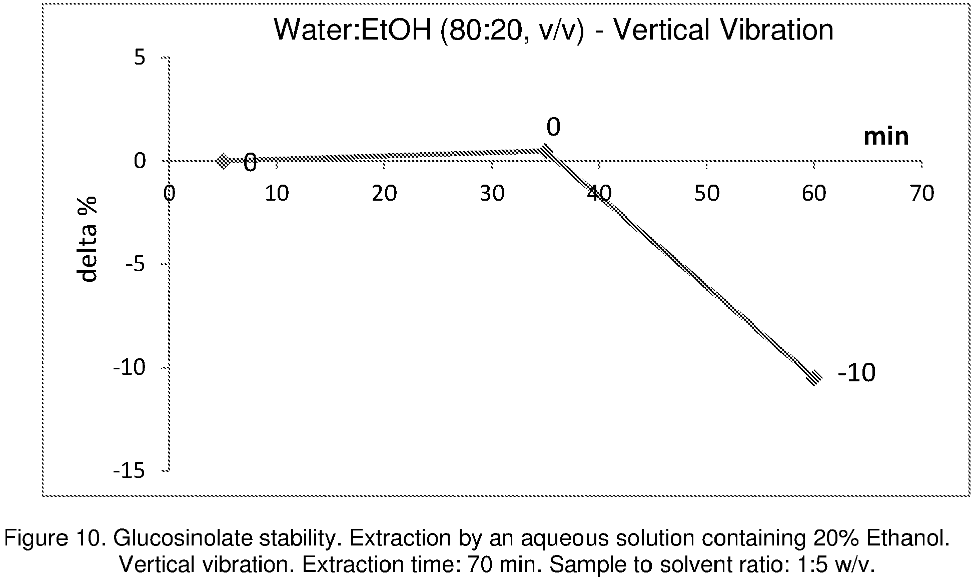

To establish the effect of the sample to solvent ratio, Moringa seeds dissolved in an aqueous solution containing 20% ethanol, sample to solvent ratio from 1:5 to 1:200 w/v, were extracted for 70 min by a vertical vibration system. At fixed times of 50 µl was withdraw, diluted to 1 ml by water and the solutions centrifuged at 1000 g for 90 sec. 20 µL of the clear solution was injected in the HPLC system. Results are showed in figure 11. The results obtained show that even with the low estration drug:solvent (1: 5) tested, glucosinolates were exhaustively extracted from the matrix.

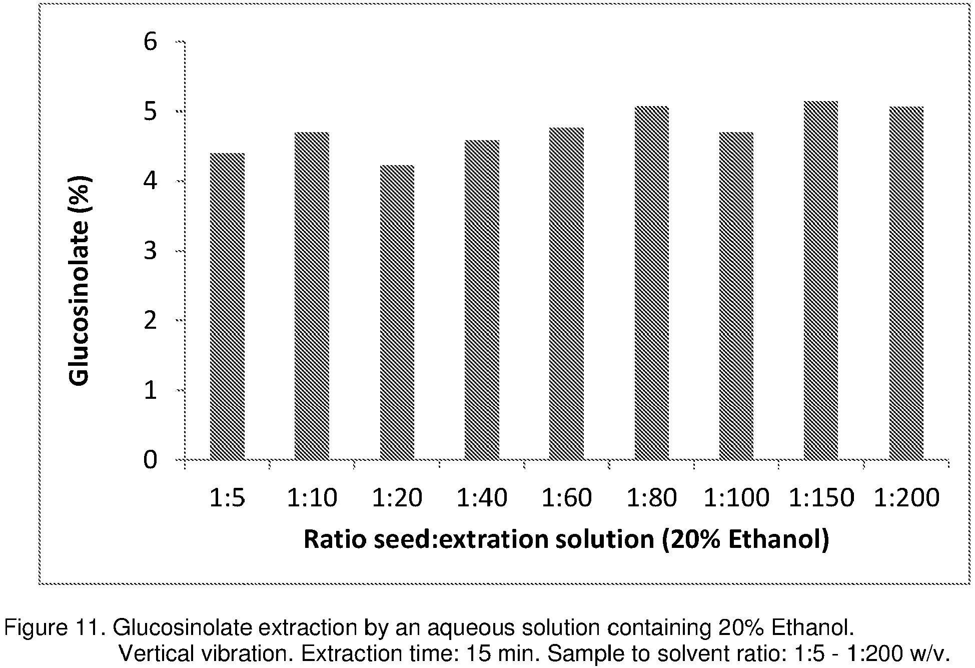

### 3.4 Seeds extraction by hot water.

It seems that in the applied conditions the glucosinolates was not stable.Indeed, after 60 min about 63% of the glucosinolates disappeared.

The instability of the glucosinales was due to the activity of the mirosinose, an enzyme that convert glucosinolate to isothiocyanate. Thus, to enhance glucosinolate stability, seed were treated with water at different temperature. In particular, seeds were extracted by hot water with temperature in the range 80-105 °C. To establish the effect of the temperature to glucosinolates stability, Moringa seeds were extracted in hot water at 80, 90, 95 and 100 °C. At fixed times, range 5-60 min,50 µl was withdraw, diluted to 1 ml by water and the solutions centrifuged at 1000 g for 90 sec. 20 µL of the clear solution was injected in the HPLC system to evaluate the glucosinolate percentage in the extract. The obtained results (Figure 12) show that glucosinolates were exhaustively extracted from the matrix after 10 min and in the range 90-100°C the extraction was exhaustive.

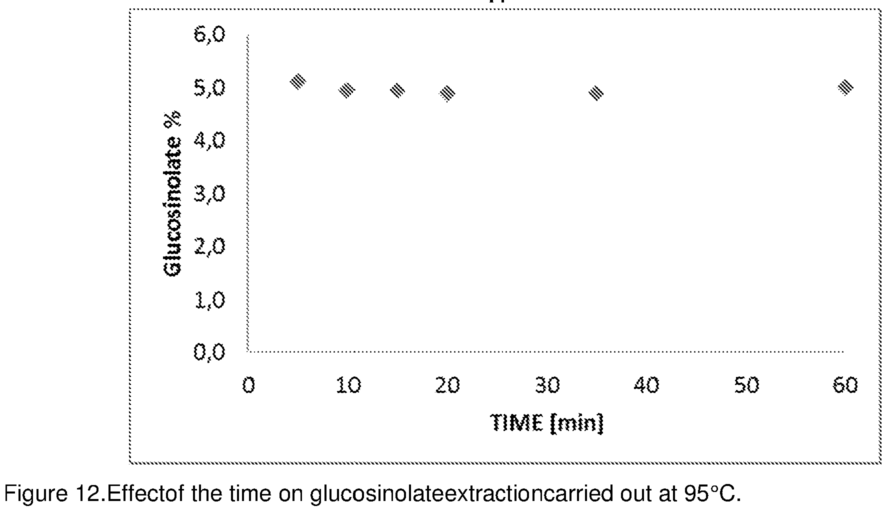

The extracts obtained at different temperature were analyzed after 20, 60, 120, 240 and 360 min evaluating the glucosinolate stability. The obtained results are reported in table 3.

**Table 3. Glucosinolatstability in the extracts obtained at different temperature.**

| Time after extraction [min] | | % | | |
|---|---|---|---|---|
| | 80°C | 90°C | 95°C | 100°C |
| 20 | 98 | 100 | 100 | 102 |
| 60 | 95 | 98 | 99 | 99 |
| 120 | 93 | 96 | 98 | 98 |
| 240 | 90 | 95 | 97 | 98 |
| 360 | 83 | 91 | 99 | 99 |

The extract obtained at 95°C was subjected to spray-dry to get the commercial product. The analysis carried out on the spray-dry extract showed that it was possible to obtain products containing amounts of glucosinolates in the range 5-40%.

### 3.5 Qualitative determination of polyphenols in Moringa leaf

The "on-line" UV and negative ion High Resolution-MS spectra (Figure 13) of leaf extractwere more complicated than those of seeds, suggesting the high complexity of the mixtures analyzed.

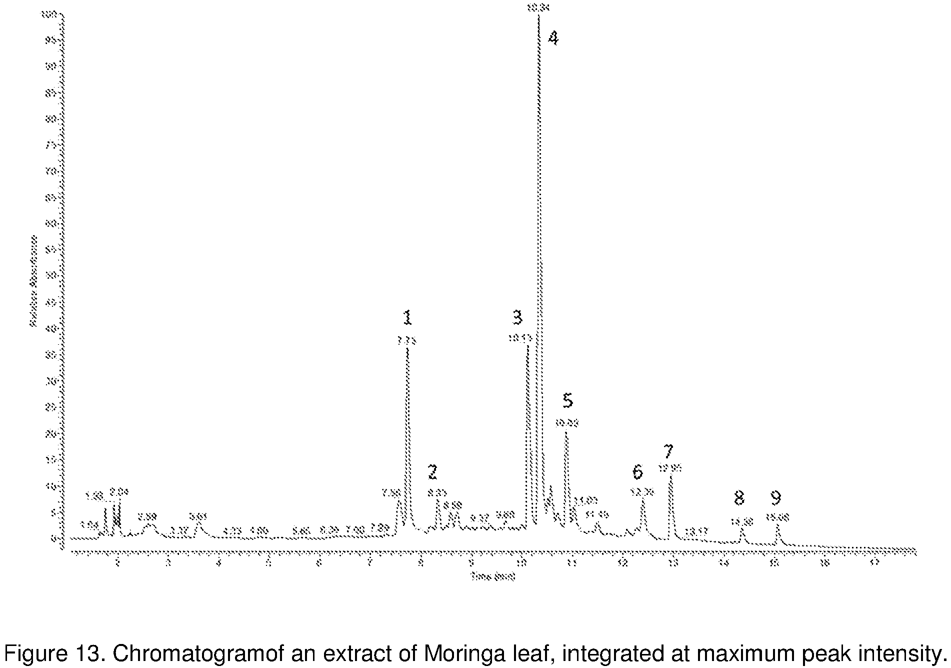

The identity of the peaks 1-9 was established by "on-line" UV spectra, molecular ion and product ions evaluation.Peak1 showed an UV spectrum suggesting a structure of 3,4-dihydroxylated-cinnamic acid derivatives and displayed [M-H]⁻ ions at (m/z)of 353.0879 u. Peak 1 showedions product with (*m*/*z*)⁻ 191.0563 u[M-H-162.0317]⁻, 179.0350u (caffeic acid) and 135.0451u (caffeic acid-H-CO₂) in its MS/MS spectra. Based on the above observations peak 1 was identified as chlorogenic acid (Caffeoyl-quinic acid). Fragmentation pattern of the peak 1 is reported in figure 14. Peak 2showed an UV spectrum suggesting a structure of 4-dihydroxylated-cinnamic acid derivatives and displayed [M-H]⁻ ions at (m/z)of 337.0932 u. Peak 2showedions product with (*m*/*z*), 191.0563 u[M-H-46.0369]⁻, 163.0401u (p-Coumaric acid) and 119.0501u (p-coumaric acid-H-CO₂) in its MS/MS spectra. Based on the above observations peak 2 was identified as acid p-Coumaroyl-quinic).

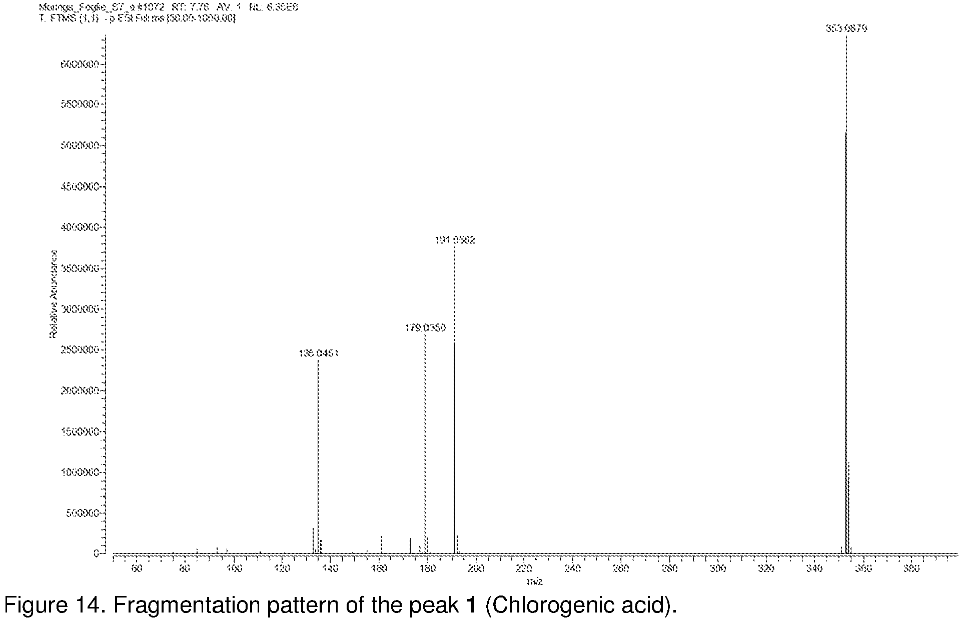

Peaks 3-9showed an UV spectrum suggesting a flavonol-structure like. In particular, Peak4 showed an UV spectrum suggesting a structure of quercetin derivative and displayed [M-H]⁻ ions at (*m*/*z*)⁻of 505.0988 u and product ions with (*m*/*z*)*⁻* 463.0884 u[M-H-42.0104]⁻ and 300.0287 u (Quercetin). Based on the above observations peak 4 was identified as Quercetin-glucoside-acetate and in figure 15 is reported its fragmentation pattern. Table 4 reports the Parent ion and fragments obtained by UPLC-HR-MS/MS of peak 1-9 in Moringa leaves extract.

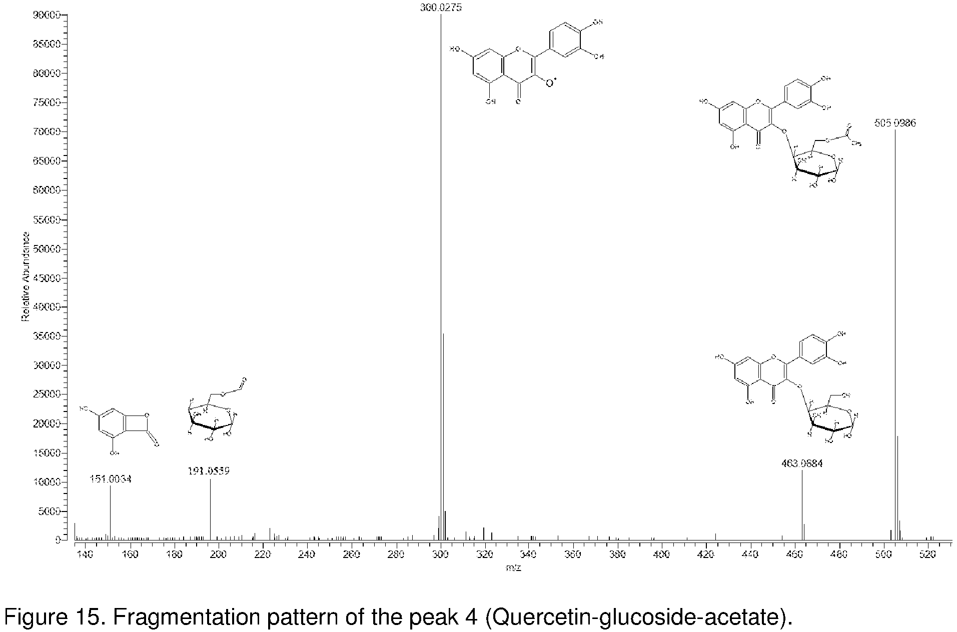

**Table 4. Parent ion and fragments obtained by UPLC-HR-MS/MS of peak 1-9 in Moringa leaves extract.**

| Peak | [M-H]⁻ | Fragments[M-H]- | Compound |
|---|---|---|---|
| 1 | 353.0880 | 191.0563, 179.0351, 135.0452 | Chlorogenic acid |
| 2 | 337.0932 | 191.0563, 163.0401, 119.0501 | p-Coumaroyl-quinic |
| 3 | 463.0886 | 300.0287 | Quercetin-glucoside |
| 4 | 505.0988 | 463.0884, 300.0278 | Quercetin-glucoside-acetate |
| 5 | 447.0935 | 284.0331 | Kaempferol-glucoside |
| 6 | 489.1040 | 284.0331 | Kaempferol-glucoside-acetate |
| 7 | 477.1038 | 314.0432 | Isorhamnetin-glucoside |
| 8 | 519.1144 | 314.0432 | Isorhamnetin-glucoside-acetate |
| 9 | 300.0287 | 151.0400 | Quercetin |

### 3.6 Quantitative determination of polyphenols in Moringa leaf

Quercetin-3-glucoside working solutions were in the range 5-100 µg/mL and the calibration curve is reported in figure 16. The flavonoid percentage in the analyzed samples is reported in table 5.

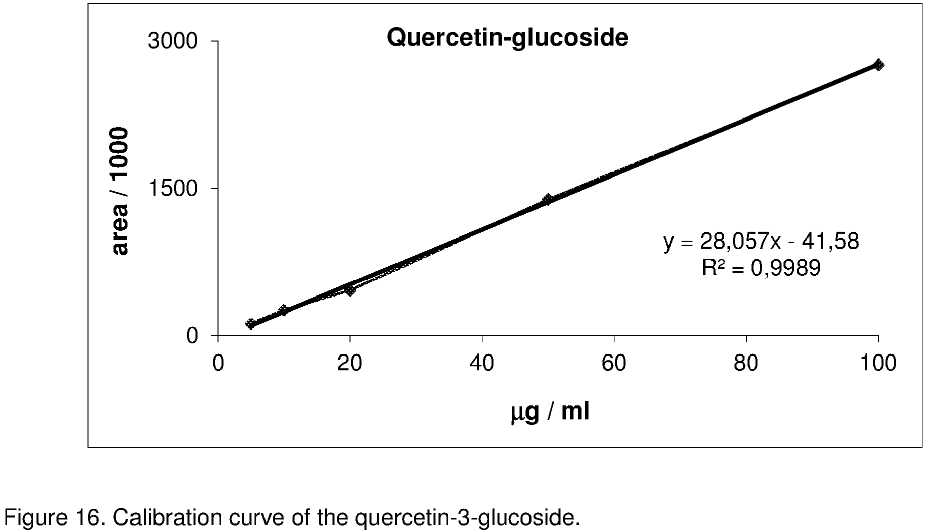

**Table 5. Flavonoid percentage in the samples of Moringa leaves.**

| **Sample** | **Description** | **% Flavonoidi** |
|---|---|---|
| 4 | Leaf | 0.03 |
| 5 | Leaf | 1.16 |
| 6 | Leaf | 1.18 |
| 7 | Leaf | 1.10 |
| 8 | Leaf | 1.59 |
| 9 | Leaf | 0.22 |
| 10 | Leaf | 1.10 |
| 11 | Leaf | 1.37 |
| 12 | Leaf | 1.36 |

### 3.7 Recovery of flavonoids in Moringa leaf extracts

Moringa leaves were extracted with aqueous solutions containing 0-20-40-60-80 and 100% ethanol and results are showed in figure 17. It seems that the solutions containing water or low percentages of ethanol (e.g. 20%, v/v) were effective in extracting quantitatively glucosinolates and flavonoids from seeds and leaves, respectively. To increase the shelf life of the extractswas preferable using solutions at least containing 20% ethanol.

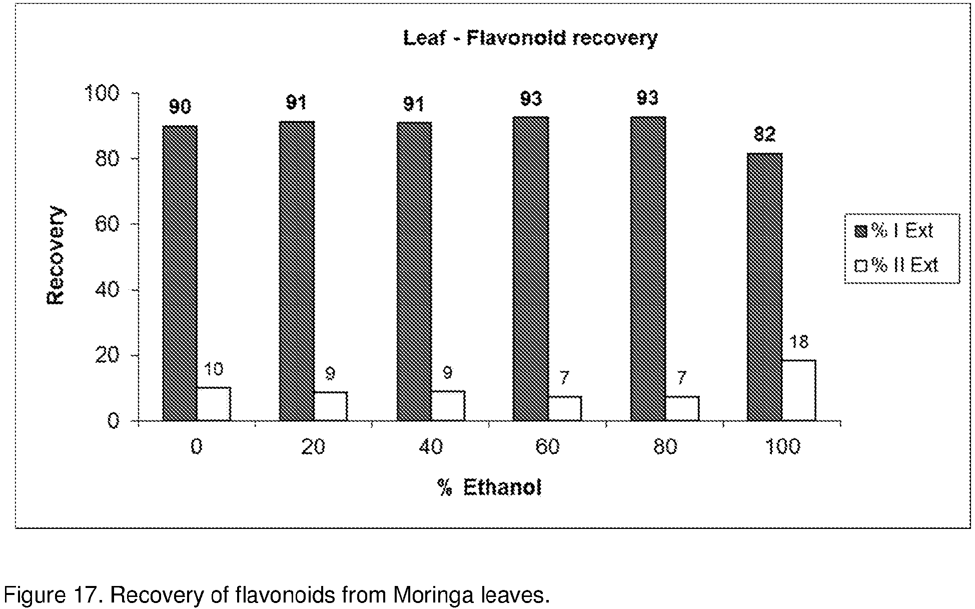

### 3.7 Protein in Moringa leaf and seed

The amount of protein found in leaves and seeds was 33±4 and 22±3%, respectively. To highlight that approximately 60 and 55% protein was found in the residue after extraction of seeds and leaves, respectively. The values obtained are comparable to those reported in the literature (Leone et al., 2015, 2016)

### 4. Conclusion

UPLC-Orbitrap-ESI-MS fingerprints of seed and leaf revealed the presence of glucosinolates and flavonoids, respectively. Furthermore, the use of the MS full spectra in high-resolution is a potentially useful and effective tool to determine active compounds in *Moringa oleifera* tissues. Moringa seed is a very rich source of glucosinolates, in particular of Glucocosinalbin, an uncommon member of the glucosinolate family with promising antimicrobial and anticarcinogenic properties. Finding of phenolic compounds in leaf is interesting because they are active antioxidant components of *Moringa oleifera* responsible for its anti-inflammatory, atherosclerotic and antidiabetic activities.

### Extraction may carried out in hot water at 95±5 °C for 5-20 min. In these conditions the extract was stable for at least 360 min

Regarding protein, results obtained in this study showed that M. *oleifera* leaves and seeds might be used as sources of low-cost protein in nutritional applications such as food supplements. Seed flour had higher protein content than those of several legumes.Therefore, M. *oleifera* seeds and leaves have good potential as nutritional supplements.In addition, most of the protein seems not present in aqueous extracts of seeds and leaves. Thus, the residue of the extraction must be considered a good source of protein.

### Bibliography

- Adam HM, Nur KAD, Dyaningtyas P, et al. (2012) Ethanolic extract of Moringa oleifera increased cytotoxic effect of doxorubicin on HeLa cancer cells. J. Nat. Remedies, 12, 108-4.
- Amaglo NK, Bennett RN, Lo C, et al. (2010) Profiling selected phytochemicals and nutrients in different tissues of the multipurpose tree Moringa oleifera L., grown in Ghana. Food Chem. 122, 1047-54.
- Brunelli DA, Tavecchio M, Falcioni C, et al. (2010) The isothiocyanate produced from glucomoringin inhibits NF-kB and reduces myeloma growth in nude mice in vivo. Biochem Pharmacol, 79, 1141-8.
- Forster N, Ulrichs C, Schreiner M, et al. (2015) Development of a reliable extraction and quantification method for glucosinolates in Moringa oleifera. Food Chem. 166, 456- 64.
- Karim NAA., Ibrahim MD, Kntayya SB, Rukayadi Y, Hamid HA, Razis AFA. (2016) Moringa Oleifera Lam: Targeting Chemoprevention. Asian Pac. J. Cancer Prev. 17, 3675-3686
- Leone A, Spada A, Battezzati A, et al. (2016) Moringa oleifera seeds and oil: Characteristics and uses for human health. Int. J. Mol. Sci. 17(12). pii: E2141
- Leone A, Fiorillo G, Criscuoli F, et al. (2015) Nutritional characterization and phenolic profiling of Moringa oleifera leaves grown in Chad, Sahrawi refugee camps, and Haiti. Int. J. Mol. Sci. 16(8):18923-37.
- Maldini M, Maksoud SA, Natella F, Montoro P, Petretto GL, Foddai M, De Nicola GR, Chessa M. Pintore G. (2014) Moringa oleifera: study of phenolics and glucosinolates by mass spectrometry. J. Mass Spectrom. 49, 900-910.
- Mbikay M. (2012) Therapeutic potential of Moringa oleifera leaves in chronic hyperglycemia and dyslipidemia: a review. Front. Pharmacol 3: 24
- Razis AFA, Ibrahim MD, Kntayya SB. (2014) Health benefits of Moringa oleifera. Asian Pac. J. Cancer Prev. 15, 8571-8576
- Stohs SJ, Hartman MJ. (2015) Review of the safety and efficacy of Moringa oleifera. Phytother. Res. 29: 796-804
- Teixeira EMB, Carvalho MRB, Neves VA, Silva MA, Arantes-Pereira L. (2014) Chemical characteristics and fractionation of proteins from Moringa oleifera Lam. leaves. Food Chem. 147, 51-54.
- Vongsak B, Sithisam P, Gritsanapan W. (2014) Simultaneous HPLC quantitative analysis of active compounds in leaves of Moringa oleifera Lam. J. Chromatogr. Sci. 52, 641-645.
- World Health Organization. (1985) Energy and protein requirements. Report of a Join FAO/WHO/UNU Expert Consultation Meeting Series, n.724, Geneva, Switzerland.

## Claims

1. Method for realization of dried extracts of *Moringa oleifera* seed and leaves to be used for nutraceutical products or supplements, comprising the following steps:
- providing *Moringa oleifera* plant seeds and leaves and grinding said seeds and leaves so as to obtain a fine powder;
- treating said powder with aqueous solution at fixed temperature, said solution containing water and ethanol so as to obtain extracts comprising standardized and stables Glucosinolates, Polyphenols and Proteins; and
- subjecting said extracts to a spray-drying treatment,
wherein said fixed temperature is between 95 and 100°C.

2. Method according to claim 1, wherein said aqueous solution contains at least 20% ethanol.

3. Nutraceutical product comprising using dried extracts obtained by method according to claims 1 or 2.

4. Nutraceutical supplement comprising dried extracts of *Moringa oleifera* obtained by method according to claims 1 or 2.

## Patentansprüche

1. Verfahren zur Herstellung von getrockneten Extrakten aus Samen und Blättern von *Moringa oleifera* zur Verwendung für nutrazeutische Produkte oder Ergänzungsmittel, umfassend die folgenden Schritte:
- Bereitstellen von Pflanzensamen und Blättern von *Moringa oleifera* und Mahlen der Samen und Blätter, so dass ein feines Pulver erhalten wird;
- Behandeln des Pulvers mit wässriger Lösung bei festgelegter Temperatur, wobei die Lösung Wasser und Ethanol enthält, so dass Extrakte erhalten werden, die standardisierte und stabile Glucosinolate, Polyphenole und Proteine umfassen; und
- Unterziehen der Extrakte einer Sprühtrocknungsbehandlung,
wobei die festgelegte Temperatur 95 bis 100 °C beträgt.

2. Verfahren gemäß Anspruch 1, wobei die wässrige Lösung mindestens 20% Ethanol enthält.

3. Nutrazeutisches Produkt umfassend getrocknete Extrakte, die durch das Verfahren gemäß den Ansprüchen 1 oder 2 erhalten werden.

4. Nutrazeutisches Ergänzungsmittel umfassend getrocknete Extrakte von *Moringa oleifera,* die durch das Verfahren gemäß den Ansprüchen 1 oder 2 erhalten werden.

## Revendications

1. Procédé d'obtention d'extraits secs de gousses et de feuilles de *ben oléifère* à utiliser pour des produits ou des suppléments nutraceutiques, comprenant les étapes suivantes consistant à :
- fournir des gousses et des feuilles de *ben oléifère* et broyer lesdites gousses et feuilles de manière à obtenir une poudre fine ;
- traiter ladite poudre avec une solution aqueuse à température fixe, ladite solution contenant de l'eau et de l'éthanol de manière à obtenir des extraits comprenant des Glucosinolates, des Polyphénols et des protéines normalisés et stables ; et
- soumettre lesdits extraits à un traitement de séchage par atomisation,
dans lequel ladite température fixe est comprise entre 95 et 100°C.

2. Procédé selon la revendication 1, dans lequel ladite solution aqueuse contient au moins 20 % d'éthanol.

3. Produit nutraceutique comprenant des extraits secs obtenus par un procédé selon les revendications 1 ou 2.

4. Complément nutraceutique comprenant des extraits secs de *ben oléifère* obtenus par un procédé selon les revendications 1 ou 2.
